# DEMANDE DE BREVET EUROPEEN

(11) **EP 4 298 910 A1**
(43) Date de publication de la demande: **03.01.2024**
(21) Numéro de dépôt: 22305955.1
(22) Date de dépôt: 30.06.2022
(51) Int. Cl.: A01N 63/40, A01P 1/00, A61K 9/50, A61K 35/76, A61K 47/06

(54) **MICROCAPSULE COMPRENANT UN BACTÉRIOPHAGE**

(71) Demandeur: Lesaffre et Compagnie, 75001 Paris (FR); Institut national de recherche pour l'agriculture, l'alimentation et l'environnement, 75007 Paris (FR)
(72) Inventeur: BOYER, Mickaël, 59700 Marcq en Baroeul (FR); DREVELLE, Antoine, 33000 Bordeaux (FR); LOPEZ FERNANDEZ, Juan Sebastian, 75013 Paris (FR); PROFIZI, Camille, 44300 Nantes (FR); TORRES BARCELÓ, Clara, 34000 Montpellier (FR)
(74) Mandataire: Lavoix

(57) **Abrégé**

La présente invention est relative au domaine de la protection contre les maladies infectieuses bactériennes au moyen de compositions comprenant des bactériophages. Elle concerne en particulier une composition antibactérienne comprenant :au moins un bactériophage choisi parmi les *Podoviridae,* les *Myoviridae* et les *Siphoviridae,* à titre d'agent actif, dans un cœur liquide, une coque gélifiée encapsulant le cœur liquide comprenant ledit bactériophage et comprenant des particules colloïdales de filtre UV dispersées dans laquelle le filtre UV n'est pas un composé soluble antioxydant.

## Description

La présente invention est relative au domaine de la protection contre les maladies infectieuses bactériennes au moyen de compositions comprenant des bactériophages.

Les bactéries pathogènes sont susceptibles d'infecter divers hôtes : humain, animal, plante. Les antibactériens sont susceptibles de traiter les infections bactériennes, soit en éliminant les bactéries (composés bactéricides), soit en bloquant leur multiplication (composés bactériostatiques). Schématiquement, les antibactériens peuvent être des antibiotiques d'origine végétale ou fongique, d'origine bactérienne, ou d'origine fossile (par extraction ou synthèse chimique). Les antibactériens peuvent aussi être d'origine animale ou encore d'origine virale. Dans ce dernier cas, il s'agit des phages ou bactériophages qui sont des virus infectant les bactéries.

Les bactéries ont la capacité de muter et ainsi de s'adapter à leur environnement et aux antibactériens. Pour cette raison, la résistance aux antibiotiques est devenue un problème majeur et la prise de conscience de cette résistance conduit à la prise en compte de traitements antibactériens alternatifs comme les bactériophages. En outre, les bactériophages s'adaptent rapidement aux mutations bactériennes sans pour autant avoir un effet négatif sur la flore commensale. Enfin, les bactériophages n'infectent pas les cellules eucaryotes et sont donc inoffensifs vis-à-vis des humains, animaux, poissons ou plantes sur lesquels ils peuvent être utilisés.

Dans le domaine de la santé humaine, l'administration de bactériophages dès les années 1960 (par exemple le phage ϕX174, un *Micoviridae* infectant *Escherichia coli*) a permis de démontrer leur innocuité, de les utiliser plus particulièrement en cas d'antibiorésistance ou encore en complément d'une antibiothérapie. De façon non exhaustive, il est possible de citer les infections de la sphère ORL à *Pseudomonas aeruginosa* comme des otites, et les infections de la sphère intestinale à *Escherichia coli.*

Dans le domaine de la nutrition et santé animale où la tendance est à la réduction de l'utilisation des antibiotiques, l'intérêt pour l'utilisation des bactériophages est réel. Par exemple en pisciculture pour lutter contre les pathogènes de la truite arc-en-ciel ou du saumon tel *Flavobacterium psychrophilum,* ou bien contre *Yersinia ruckeri* qui infecte le saumon. Pour les élevages de volaille, des cocktails de phages sont utilisés contre *Salmonella* ou *Clostridium perfringens,* tels que ceux développés par Intralytix Inc.

Dans le domaine de la santé des plantes dans lequel les infections bactériennes sont responsables de pertes économiques importantes, la majorité des solutions de biocontrôle existantes sont basées sur l'utilisation de cuivre dont la toxicité et les effets secondaires potentiels conduisent à rechercher d'autres solutions de biocontrôle parmi lesquelles les bactériophages.

Enfin, dans le domaine de l'agro-alimentaire, les bactériophages peuvent être utilisés dans la maitrise de la sécurité des aliments, par leur capacité à limiter les développements des pathogènes dans les étapes de transformation des aliments ou sur les surfaces en contact avec les aliments. De manière non exhaustive, *Listeria, Shigella, Salmonella, Escherichia coli* ou *Campylobacter jejuni* sont des cibles prioritaires de l'industrie agroalimentaire.

L'une des difficultés lors de l'emploi des bactériophages est leur fragilité, leur sensibilité à l'environnement. En effet, ils présentent des parois constituées uniquement de protéines, parois qui sont plus fines et plus fragiles que celles des bactéries. Plus particulièrement, le pH extrême de certains sols ou encore les radiations UV, par exemple des rayons du soleil, sont des facteurs à prendre en compte.

Améliorer la stabilité et le maintien de la viabilité des phages dans le temps est un défi de taille pour envisager leur utilisation à large échelle. Des technologies de séchage par pulvérisation (spray-drying) ou congélation (freeze drying) existent (Matinkhoo et al., 2011, J Pharm Sci ;100(12):5197-205 ; Jones et al., 2012, Bacteriophage 2: 208-214; Vandenheuvel et al., 2013, Eur J Pharm Biopharm ; 84(3):578-82; Leung et al., 2016, Pharmaceutical research, 33(6), 1486-1496 ; Leung et al., 2017, International journal of pharmaceutics, 521(1-2), 141-149). Des technologies d'encapsulation appelées à améliorer la stabilité durant le stockage, le transport et l'application ont été testées.

Dans sa recherche de solutions destinées à protéger les bactériophages des agressions extérieures de type pH ou rayons UV pendant le stockage ou l'application, la Demanderesse a expérimenté le co-séchage de bactériophages et de levures, comme expliqué dans la demande FR2110984.

De façon surprenante, l'encapsulation de bactériophages, utilisés en qualité d'agent actif, dans une coque comprenant des particules de type colloïdal de filtre UV dispersé, permet de conserver la viabilité des phages et leur activité antibactérienne. Les phages ainsi encapsulés permettent de contrôler efficacement les pathogènes bactériens, avantageusement les pathogènes bactériens dirigés contre les humains, les animaux ou les plantes.

Ainsi, l'invention porte sur une composition antibactérienne comprenant au moins un bactériophage choisi parmi les *Podoviridae,* les *Myoviridae* et les *Siphoviridae,* à titre d'agent actif dans un cœur liquide, ledit cœur liquide comprenant le bactériophage étant encapsulé dans une coque gélifiée comprenant des particules colloïdales de filtre UV dispersées, dans laquelle le filtre UV n'est pas un composé soluble antioxydant.

### [Description détaillée]

Les termes phages ou bactériophages seront employés de façon indifférente. Les phages ou bactériophages sont des virus qui infectent des bactéries et ce, de façon très spécifique et sans effet secondaire significatif sur l'hôte, humain, animal ou végétal. Ainsi, la composition antibactérienne objet de la présente invention est dirigée contre toute bactérie potentiellement pathogène, que l'hôte soit un humain, un animal ou une plante. Par activité antibactérienne, on comprend une activité lytique de la part du bactériophage sur la bactérie suite à l'infection de la bactérie par celui-ci.

Selon un mode de réalisation, les pathogènes ciblés sont des bactéries susceptibles d'infecter les plantes et/ou les produits de récoltes. Les maladies bactériennes des plantes causent des pertes très importantes dans les cultures agricoles et cultures de légumes. Peu de traitements existent, beaucoup sont à base de cuivre. Iriarte et al. (Bacteriophage, 2012 ; 2 (4) : 215-224) ont montré que le phage peut pénétrer dans la plante et se déplacer des racines vers les feuilles en persistant dans la plante jusqu'à 7 jours.

Pour les maladies des parties aériennes des plantes, les pathogènes ciblés sont choisis parmi : *Erwinia spp.* ou plus spécifiquement *Erwinia amylovora, Xanthomonas spp.,* ou plus spécifiquement *Xanthomonas axonopodis var. citri* dont l'hôte est le citrus et responsable de la maladie communément appelée *Citrus greening, Xylella spp.* ou plus spécifiquement *Xylella fastidiosa,* susceptible d'infecter environ 600 espèces végétales appartenant à plus de 80 familles botaniques différentes : vigne, agrumes, arbres fruitiers, amandier, olivier, cerisier, caféier, avocatier, luzerne, laurier-rose, chêne, érable etc. dont la maladie qui touche la vigne est appelée maladie de Pierce.

Pour les maladies du sol, pour lesquelles une application au goutte à goutte ou au niveau de la semence est nécessaire, les pathogènes ciblés sont choisis parmi *Pseudomonas* spp. ou plus spécifiquement *Pseudomonas syringae* pv. *tomato* dont l'hôte est le plant de tomate et responsable de la maladie appelée *bacterial speck* (tâche bactérienne ou moucheture), *Ralstonia* spp. ou plus spécifiquement *Ralstonia solanacearum* dont l'hôte est également le plant de tomate et responsable de la maladie appelée *bacterial wilt* (flétrissure bactérienne).

Pour les produits de récoltes de plants végétaux, pour lesquels l'application se fait directement sur le produit, les pathogènes particulièrement ciblés sont les espèces *Pectobacterium* et *Dickeya* susceptibles d'infecter la pomme de terre.

*Erwinia amylovora* est une bactérie pathogène de plante responsable du feu bactérien qui est une maladie contagieuse affectant les pommiers, les poiriers et d'autres plantes de la famille des *Rosacae* (Momol MT; Aldwinckle HS, 2000. Genetic diversity and host range of Erwinia amylovora. In: Vanneste JL, ed. Fire blight the disease and its causative agent, Erwinia amylovora. Wallingford, Oxon UK: CABI Publishing, 55-72 ; Zwet T van der; Keil HL, 1979. Fire blight, a bacterial disease of Rosaceous plants. Agriculture Handbook, Science and Education Administration USDA. Beltsville, USA:USDA, No. 510:200 pp). Un criblage d'une collection de 33 phages infectant l'espèce *Erwinia* a par exemple permis d'isoler cinq phages de la famille des *Myoviridae* qui sont susceptibles d'infecter un large éventail de souches bactériennes d'*Erwinia amylovora*. Lesdit phages, appelé ci-après respectivement BPH1, 31Dx, 26C, P2 et 2By, ont été utilisés pour les tests d'encapsulation de compositions antibactériennes selon l'invention.

Selon un mode de réalisation, les pathogènes ciblés sont des bactéries susceptibles d'infecter les humains. Par exemple, de façon non exhaustive, des souches de bactéries choisies parmi *Escherichia coli, Listeria monocytogenes, Campylobacter jejuni, Staphylococcus aureus, Clostridium perfringens* ou des souches du genre *Salmonella.*

Selon un mode de réalisation, les pathogènes ciblés sont des bactéries susceptibles d'infecter des animaux, tels *Escherichia coli, Salmonella, Campylobacter, Staphylococcus, Flavobacterium psychrophilum, Yersinia ruckeri, Clostridium perfringens.* Les animaux à traiter sont aussi bien des bovins que des ovins, des porcs, des volatiles, des poissons, des crustacés etc.

Selon un mode de réalisation, les pathogènes ciblés sont des pathogènes contre lesquels l'industrie agro-alimentaire doit se prémunir, pour les produits laitiers, de viande ou de poisson : *Listeria, Salmonella, Shigella, Escherichia coli, Campylobacter jejuni* etc.

De manière avantageuse, l'encapsulation permet de conserver à la composition antibactérienne selon l'invention ses propriétés antibactériennes, telles que le démontrent les résultats expérimentaux rapportés dans les exemples ci-après. En effet, pour démontrer la viabilité d'un phage et dénombrer les phages, il est indispensable que ledit phage reste efficace contre le pathogène contre lequel il est dirigé.

Les capsules utilisées dans la composition antibactérienne selon l'invention se présentent comme une solution liquide comprenant un biopolymère ayant des propriétés gélifiantes et une coque de particules colloïdales de filtre UV dispersées, dans lequel le filtre UV n'est pas un composé soluble antioxydant.

Les microcapsules utilisées dans la composition antibactérienne selon l'invention permettent de confiner les bactériophages sans les immobiliser.

Les microcapsules sont bien connues de l'homme du métier. Elles peuvent être formées par différentes techniques et avoir différentes compositions de coque. Typiquement les microcapsules utilisées dans le cadre de l'invention sont produites selon le procédé de fabrication décrit dans le brevet d'invention Français n°2939012 et la demande internaitonaleWO2010063937 A1.

Elles sont formées d'un cœur liquide comprenant au moins un phage constituant l'agent actif de la composition antibactérienne selon l'invention. Ce cœur liquide peut comprendre un mélange de différents phages.

Le cœur liquide est encapsulé par une enveloppe gélifiée sensiblement solide appelée la coque. En particulier, ladite coque est solide. De préférence, la coque des microcapsules est principalement composée d'un biopolymère ayant des propriétés gélifiantes. En particulier, ladite coque est composée dudit biopolymère ayant des propriétés gélifiantes. Ce biopolymère en proportion majoritaire dans la coque est le biopolymère principal. De tels biopolymères ayant des propriétés gélifiantes sont par exemple l'alginate, la gomme de gellane, la gomme de xanthane, la pectine, le chitosan, l'agar ou le carraghénane. De préférence, la coque des microcapsules formant la composition antibactérienne selon l'invention est principalement composée d'alginate, en particulier ladite coque est composée d'alginate.

Lorsqu'elles sont sous forme hydratée, comme par exemple en suspension dans une solution aqueuse, les microcapsules de la composition antibactérienne selon l'invention ont un diamètre moyen compris entre 50 et 4000 µm, de préférence compris entre 100 et 2000 µm, plus particulièrement entre 200 et 1000 µm, avantageusement entre 200 et 600 µm. Ce diamètre moyen peut être mesuré par diverses techniques bien connues de l'homme du métier tel que la granulométrie basée sur la diffraction de la lumière laser, le fractionnement par tamisage ou l'imagerie par microscopie optique. Elles peuvent également se présenter sous forme déshydratée. De préférence, la déshydratation est partielle, et les microcapsules déshydratées ont de préférence un taux d'humidité inférieur à 10%, mesuré par un analyseur d'humidité après déshydratation.

Par particules colloïdales de filtre UV, on entend des particules ayant une taille moyenne comprise entre 10 nm et 10 µm, plus préférentiellement entre 100 nm et 5 µm. En général, plus les particules sont petites, plus elles seront efficaces contre les UV. Ces filtres peuvent aussi permettre d'opacifier les microcapsules.

Dans le contexte de la présente invention, le filtre UV n'est pas un composé soluble anti-oxydant. En effet, ces composés ont tendance à se dégrader plus rapidement lorsqu'ils sont exposés aux UV. Le filtre UV utilisé dans les microcapsules formant la composition antibactérienne selon l'invention peut être organique ou inorganique, ou encore un mélange de filtre organique et inorganique. Ledit filtre UV est choisi de préférence parmi l'oxyde de titane, le noir de carbone, le biochar, le charbon de bois, le latex, la silice, les argiles, et leurs mélanges. Le biochar est le produit de la pyrolyse de biomasse, utilisé comme amendement de sols agricoles. Les argiles utilisées comme filtres UV sont par exemple le talc ou le kaolin et leurs mélanges. Le kaolin est un type d'argile aussi utilisé pour protéger certaines cultures des bioagresseurs en agriculture.

De préférence, le filtre UV utilisé dans les microcapsules formant la composition antibactérienne selon l'invention est biodégradable et/ou comestible et/ou pharmaceutiquement acceptable.

Par « particules de filtre UV dispersées », on entend des particules dispersées, de préférence dans la coque de la microcapsule, ceci afin de permettre un effet anti-UV homogène sur la surface de la microcapsule.

L'invention concerne également un procédé de prévention et/ou de traitement de plants végétaux contre au moins une bactérie pathogène comprenant les étapes de :
fournir une composition antibactérienne comprenant au moins un bactériophage au titre d'agent actif contre ladite au moins une bactérie pathogène, ledit bactériophage étant protégé contre les UV dans une microcapsule comprenant des particules colloïdales de filtre UV dispersées dans la coque, dans laquelle le filtre UV n'est pas un composé soluble antioxydant,
appliquer ladite composition sur les parties aériennes du plant végétal.

En particulier, ledit au moins un bactériophage est choisi parmi les *Podoviridae,* les *Myoviridae* et les *Siphoviridae.*

Selon un mode de réalisation, les produits de récolte desdits plants végétaux sont soumis à la prévention et/ou au traitement antibactérien.

Selon un mode de réalisation, l'application de la composition antibactérienne peut aussi être effectuée par épandage sur le sol. Selon un autre mode de réalisation, l'application de la composition antibactérienne peut être effectuée autour des semences, en particulier mais pas seulement par enrobage ou pelliculage. Selon un autre mode de réalisation, l'application peut être effectuée sur les produits après récolte.

Dans un autre mode de réalisation, l'invention concerne l'utilisation d'une composition antibactérienne selon l'invention pour la nutrition et/ou l'alimentation animale et/ou humaine. Dans ce mode de réalisation, les phages utilisés ne sont pas, de préférence, des phages thérapeutiques. Autrement dit, lesdits phages ont un intérêt d'un point de vue nutritionnel ou alimentaire mais ne permettent pas de prévenir ou de traiter des maladies du sujet qui les consomme.

Par « alimentation », on entend la prise d'aliment habituelle ou fréquente. En effet, la composition antibactérienne peut contenir des phages variés qui permettent d'améliorer les propriétés d'un produit alimentaire, parmi lesquelles sa biodisponibilité ou sa durée de conservation.

Par « nutrition », on entend la prise, souvent plus ponctuelle ou lors de cure, de complément alimentaire typiquement pour éviter ou combler une carence. En effet, la composition antibactérienne selon l'invention peut contenir des phages variés qui permettent d'améliorer les apports en nutriments en améliorant leur biodisponibilité ou encore de modifier la flore intestinale.

L'invention concerne également une composition pharmaceutique comprenant une composition antibactérienne selon l'invention.

La présente invention concerne également la composition antibactérienne ou la composition pharmaceutique selon l'invention pour son utilisation comme agent antibactérien, en particulier comme médicament, et plus particulièrement pour le traitement d'une maladie causée par une bactérie, chez un sujet qui en a besoin.
Dans ce mode de réalisation, la composition antibactérienne selon l'invention comprend avantageusement un phage choisi parmi les bactériophages ciblant des bactéries pathogènes de l'Homme ou de l'animal, et plus particulièrement un bactériophage ciblant des bactéries choisies parmi *Escherichia coli, Listeria monocytogenes, Campylobacter jejuni, Staphylococcus aureus, Clostridium perfringens* ou des souches du genre *Salmonella.*

Le terme "composition pharmaceutique" tel que défini ici désigne un mélange ou une solution comprenant au moins un agent thérapeutique à administrer à un sujet afin de prévenir ou traiter une maladie particulière affectant le sujet.

Les compositions pharmaceutiques telles que définies ici comprennent donc de préférence en outre des excipients pharmaceutiquement acceptables.

Par "pharmaceutiquement acceptable", on entend ici des compositions et entités moléculaires qui ne produisent pas de réactions secondaires, allergiques ou autrement non-désirées quand elles sont administrées à un sujet.

Par "sujet", on entend ici un être vivant, de préférence un mammifère, et plus particulièrement un humain.

Par "quantité thérapeutiquement efficace", on entend ici une quantité efficace, à des doses et pendant des périodes de temps nécessaires, pour obtenir le résultat thérapeutique souhaité. Cette quantité peut varier en fonction de facteurs tels que la maladie, l'étendue de la maladie, l'âge, le sexe et le poids du sujet, et la capacité des microcapsules à provoquer un résultat thérapeutique souhaité. Une quantité thérapeutiquement efficace englobe une quantité dans laquelle tout effet toxique ou nuisible est compensé par les effets thérapeutiquement bénéfiques. Une quantité thérapeutiquement efficace englobe également une quantité suffisante pour conférer un bénéfice, par exemple un bénéfice clinique.

De telles compositions pharmaceutiques sont de préférence adaptées à la voie d'administration.

Dans un mode de réalisation particulier, les compositions pharmaceutiques sont adaptées à une administration orale, sublinguale, buccale, intranasale ou topique.

Tel que susmentionné, l'Invention concerne également une composition antibactérienne ou une composition pharmaceutique selon l'invention pour son utilisation comme médicament. L'invention concerne également une composition antibactérienne selon l'invention pour son utilisation pour la formulation de compositions pharmaceutiques.

Pour ces utilisations, la composition antibactérienne selon l'invention comprend avantageusement un phage choisi parmi les bactériophages ciblant des bactéries pathogènes de l'Homme ou de l'animal, et plus particulièrement un bactériophage ciblant des bactéries choisies parmi *Escherichia coli, Listeria monocytogenes, Campylobacter jejuni, Staphylococcus aureus, Clostridium perfringens* ou des souches du genre *Salmonella.*

En effet, l'utilisation d'une composition antibactérienne selon l'invention permet de faciliter le stockage, le conditionnement ou la préparation des compositions pharmaceutiques tout en conservant les phages actifs.

Par "traitement" ou "traiter", on entend ici atteindre, partiellement ou substantiellement, un ou plusieurs des résultats suivants : réduire partiellement ou totalement l'étendue de la maladie, améliorer un symptôme clinique ou un indicateur associé à la maladie, retarder, inhiber ou prévenir la progression de la maladie. En particulier, dans le cadre de la présente invention, la maladie à traiter est une maladie causée par une bactérie, en particulier celles susmentionnées.

Les exemples ci-après sont rapportés à titre d'illustration et ne sauraient limiter la portée de l'invention.

### Exemples

### Méthode de dénombrement des phages

Les méthodes de démonstration de la viabilité et de dénombrement des bactériophages utilisent obligatoirement les bactéries ciblées par les bactériophages testés. D'une manière générale, les méthodes de dénombrement de bactériophages de concentration inconnue sont des méthodes de bicouche en agar semi-solide connues de l'homme du métier.

Une préculture des bactéries cibles est effectuée en milieu LB liquide (bouillon Luria Bertani). Dans certains cas, l'étape de préculture en milieu liquide peut être doublée pour atteindre le volume et la concentration suffisants, ladite concentration étant évaluée par la mesure de la DO (densité optique) à 600 nm. Une dilution sériée des phages à dénombrer est effectuée en milieu LB liquide. La mise en contact des dilutions sériées de phages avec les cultures bactériennes est effectuée sur un milieu LBA (Luria Bertani Agar) en ajoutant préalablement du milieu Luria Bertani Agar semi solide à 0,75% d'agar.

La lecture et le dénombrement des plages de lyse sont effectués après une incubation de 16 à 24 heures à 30°C.

Une souche *Salmonella* Newport a été utilisée pour le dénombrement des phages anti *Salmonella* fournis par Intralytix Inc (Salmofresh^{™}).
Une souche *Listeria monocytogenes* a été utilisée pour le dénombrement des phages anti *Listeria* fournis par Intralytix Inc.
Une souche *Erwinia amylovora* Ea4 fournie par INRAe a été utilisée pour le dénombrement des phages anti*-Erwinia* (phages BPH1, 31Dx, 26C, P2 et 2By).

### Exemple 1 : test d'encapsulation d'un phage et évaluation de la stabilité après encapsulation

Le phage BPH1 susmentionné a été encapsulé dans une capsule sans filtre UV. Des essais de séchage ont été effectués avec trois supports différents de séchage. La viabilité et la stabilité dans le temps et en fonction de la température ont été évaluées.

Parmi le glycérol, le SM-sucrose et le tréhalose testés comme excipients de séchage, le SM-sucrose est celui qui a donné le rendement de séchage le plus acceptable.

La stabilité du phage encapsulé a été testée pendant un mois respectivement à +4°C, +25°C et +40°C. Le titre du phage encapsulé, sous forme liquide, diminue très peu à +4°C ou +25°C. Le titre diminue environ de 2 log à +40°C. Sous forme solide, c'est-à-dire après séchage avec le SM-sucrose comme excipient, le titre reste sensiblement le même quelle que soit la température de conservation, avec une diminution inférieure à 1 log à +40°C.

### Exemple 2 : protection des phages anti-Erwinia dans une capsule comprenant un filtre UV

Les phages BPH1, 31Dx, 26C, P2 et 2By susmentionnés ont été encapsulés dans une coque comprenant du biochar au titre de filtre anti-UV. Des phages seuls, des capsules avec ou sans biochar contenant des phages ont été exposés, sous forme liquide, aux UV. Les phages ont été déposés à la CNCM (Collection Nationale de Cultures de Microorganismes, hébergée à l'Institut Pasteur, 25 rue du docteur Roux, 75015 Paris, France) selon le traité de Budapest :
BPH1 déposé le 17 décembre 2021 sous le numéro I-5803,
31Dx déposé le 6 mai 2022 sous le numéro I-5845,
26C déposé le 6 mai 2022 sous le numéro I-5846,
P2 déposé le 6 mai 2022 sous le numéro I-5847, et
2By déposé le 6 mai 2022 sous le numéro I-5848.

Le protocole d'exposition aux rayons ultraviolets était le suivant :
récupération d'un échantillon homogène de capsules ayant une taille de 100 µm en utilisant des tamis cellulaires stériles.
échantillonnage de 3 g de capsules humides ou bien 1 g de capsules sèches, respectivement, tamisées dans une boite de Petri stérile
addition de tampon NaCl 0,1M dilué au 1/10 avec de l'eau physiologique stérile. prélèvement du témoin avant exposition
exposition aux UV avec une lampe ayant subi le préchauffage de rigueur, à une intensité de 105mW/cm².
Les boites de Petri sont placées au centre du four.
Les durées d'exposition sont respectivement de 10, 20, et 30 minutes pour les phages nus, les phages encapsulés sans filtre UV, les phages encapsulés avec filtre UV et le témoin de tampon NaCl.
Deux cents microlitres de chaque échantillon exposé aux UV sont prélevés pour le dénombrement.
Préalablement au dénombrement effectué tel qu'indiqué ci-avant, le plus possible de surnageant est enlevé, les capsules sont pesées, ouvertes avec addition de 1 mL de PBS EDTA, agitées 5 minutes sur agitateur à disque rotatif à vitesse modérée puis diluées de façon sériée pour procéder au dénombrement selon le protocole indiqué ci-dessus.

Le Tableau 1 présente les résultats de titration des phages BPH1 après des temps d'exposition aux UV respectivement de 0, 5, 10, 20 et 30 minutes. LDD signifie limite de détection.

**[Tableau 1]**

| **Echantillon** | **Tps expo (min)** | **CFU/ml** |
|---|---|---|
| témoin negatif | 0 | LDD |
| phage libre | 0 | 4,33E+07 |
| capsules humides sans biochar E1 | 0 | 2,06E+06 |
| capsules humides sans biochar E2 | 0 | 2,89E+06 |
| capsules humides avec biochar E1 | 0 | 2,89E+06 |
| capsules humides avec biochar E2 | 0 | 3,67E+06 |
| témoin negatif | 5 | LDD |
| phage libre | 5 | LDD |
| capsules humides sans biochar E1 | 5 | LDD |
| capsules humides sans biochar E2 | 5 | LDD |
| capsules humides avec biochar E1 | 5 | 4,08E+04 |
| capsules humides avec biochar E2 | 5 | 3,11E+05 |
| témoin negatif | 10 | LDD |
| phage libre | 10 | LDD |
| capsules humides sans biochar E1 | 10 | LDD |
| capsules humides sans biochar E2 | 10 | LDD |
| capsules humides avec biochar E1 | 10 | 2,06E+05 |
| capsules humides avec biochar E2 | 10 | 3,50E+05 |
| témoin negatif | 20 | LDD |
| phage libre | 20 | LDD |
| capsules humides sans biochar E1 | 20 | LDD |
| capsules humides sans biochar E2 | 20 | LDD |
| capsules humides avec biochar E1 | 20 | 1,28E+05 |
| capsules humides avec biochar E2 | 20 | 4,28E+04 |
| témoin negatif | 30 | LDD |
| phage libre | 30 | LDD |
| capsules humides sans biochar E1 | 30 | LDD |
| capsules humides sans biochar E2 | 30 | LDD |
| capsules humides avec biochar E1 | 30 | 3,61E+04 |
| capsules humides avec biochar E2 | 30 | 4,61E+04 |

Ces résultats montrent que le titre des phages diminue de façon drastique (>4log) après 5 minutes d'exposition aux UV, dans la même proportion que les phages « libres » utilisés comme contrôle.
*A contrario,* la diminution du titre est moins importante pour les phages dans des capsules avec du biochar : de 1 log après 5 minutes à 2 log après 30 minutes d'exposition). Ces résultats montrent clairement qu'une capsule comprenant du biochar protège efficacement les phages contre l'inactivation.

Des phages encapsulés et séchés, exposés aux UV après réhydratation, ont permis d'obtenir des résultats similaires après 10 minutes d'exposition, comme l'illustre le Tableau 2.

**[Tableau 2]**

| **Echantillon** | **Tps expo (min)** | **CFU/ml** |
|---|---|---|
| phage libre | 0 | 6,00E+07 |
| capsules sèches sans biochar E2 | 0 | 2,61E+06 |
| capsules sèches sans biochar E1 | 0 | 3,11E+06 |
| capsules sèches avec biochar E2 | 0 | 1,78E+06 |
| capsules sèches avec biochar E1 | 0 | 2,00E+06 |
| phage libre | 10 | 1,67E+02 |
| capsules sèches sans biochar E2 | 10 | 1,67E+02 |
| capsules sèches sans biochar E1 | 10 | 1,67E+02 |
| capsules sèches avec biochar E2 | 10 | 1,22E+06 |
| capsules sèches avec biochar E1 | 10 | 7,33E+05 |

Les Tableaux 3 et 4 présentent les résultats de titration des phages 31 Dx après des temps d'exposition aux UV respectivement de 0, 5, 10, 20 et 30 minutes. LDD signifie limite de détection, respectivement pour les capsules humides et les capsules sèches.

**[Tableau 3]**

| **échantillon** | **Temps d'exposition** | **Concentration (UFP/MI ou UFP/g)** |
|---|---|---|
| temoin negatif | 0 | 0,00E+00 |
| phage libre | 0 | 2,24E+09 |
| capsules humides E1 | 0 | 1,09E+08 |
| capsules humides E2 | 0 | 4,24E+07 |
| temoin negatif | 10 | 0,00E+00 |
| phage libre | 10 | 1,38E+05 |
| capsules humides E1 | 10 | 1,06E+07 |
| capsules humides E2 | 10 | 4,91E+06 |
| temoin negatif | 20 | 0,00E+00 |
| phage libre | 20 | LDD |
| capsules humides E1 | 20 | 3,60E+06 |
| capsules humides E2 | 20 | 2,02E+06 |
| temoin negatif | 30 | 0,00E+00 |
| phage libre | 30 | LDD |
| capsules humides E1 | 30 | 3,83E+06 |
| capsules humides E2 | 30 | 7,69E+05 |

**[Tableau 4]**

| **echantillon** | **Temps d'exposition** | **Concentration (UFP/MI ou UFP/g)** |
|---|---|---|
| temoin negatif | 0 | 1,00E+01 |
| phage libre | 0 | 5,07E+08 |
| capsules seches E1 | 0 | 3,97E+07 |
| capsules seches E2 | 0 | 3,35E+07 |
| temoin negatif | 10 | 0,00E+00 |
| phage libre | 10 | LDD |
| capsules seches E1 | 10 | 2,63E+07 |
| capsules seches E2 | 10 | 3,59E+07 |
| temoin negatif | 20 | 0,00E+00 |
| phage libre | 20 | LDD |
| capsules seches E1 | 20 | 1,76E+07 |
| capsules seches E2 | 20 | 1,60E+07 |
| temoin negatif | 30 | 0,00E+00 |
| phage libre | 30 | LDD |
| capsules seches E1 | 30 | 1,17E+07 |
| capsules seches E2 | 30 | 1,38E+07 |

Les Tableaux 5 et 6 présentent les résultats de titration des phages 26C après des temps d'exposition aux UV respectivement de 0, 5, 10, 20 et 30 minutes. LDD signifie limite de détection, respectivement pour les capsules humides et les capsules sèches.

**[Tableau 5]**

| **echantillon** | **Temps d'exposition** | **Concentration (UFP/MI ou UFP/g)** |
|---|---|---|
| temoin negatif | 0 | 0,00E+00 |
| phage libre | 0 | 1,56E+09 |
| capsules humides E1 | 0 | 7,94E+07 |
| capsules humides E2 | 0 | 8,21E+06 |
| temoin negatif | 10 | 0,00E+00 |
| phage libre | 10 | 2,07E+04 |
| capsules humides E1 | 10 | 3,27E+07 |
| capsules humides E2 | 10 | 2,89E+06 |
| temoin negatif | 20 | 0,00E+00 |
| phage libre | 20 | LDD |
| capsules humides E1 | 20 | 1,16E+07 |
| capsules humides E2 | 20 | 2,22E+06 |
| temoin negatif | 30 | 0,00E+00 |
| phage libre | 30 | LDD |
| capsules humides E1 | 30 | 1,78E+07 |
| capsules humides E2 | 30 | 1,72E+06 |

**[Tableau 6]**

| **echantillon** | **Temps d'exposition** | **Concentration (UFP/MI ou UFP/g)** |
|---|---|---|
| temoin negatif | 0 | 0,00E+00 |
| phage libre | 0 | 1,50E+09 |
| capsules seches E1 | 0 | 1,18E+08 |
| capsules seches E2 | 0 | 9,47E+08 |
| temoin negatif | 10 | 0,00E+00 |
| phage libre | 10 | 9,07E+04 |
| capsules seches E1 | 10 | 1,94E+07 |
| capsules seches E2 | 10 | 1,13E+08 |
| temoin negatif | 20 | 0,00E+00 |
| phage libre | 20 | LDD |
| capsules seches E1 | 20 | 4,45E+08 |
| capsules seches E2 | 20 | 2,50E+08 |
| temoin negatif | 30 | 0,00E+00 |
| phage libre | 30 | LDD |
| capsules seches E1 | 30 | 4,68E+07 |
| capsules seches E2 | 30 | 4,69E+07 |

Les Tableaux 7 et 8 présentent les résultats de titration des phages P2 après des temps d'exposition aux UV respectivement de 0, 5, 10, 20 et 30 minutes. LDD signifie limite de détection, respectivement pour les capsules humides et les capsules sèches. NA signifie non applicable (donnée non disponible pour des raisons techniques).

**[Tableau 7]**

| **echantillon** | **Temps d'exposition** | **Concentration (UFP/MI ou UFP/g)** |
|---|---|---|
| temoin negatif | 0 | 0,00E+00 |
| phage libre | 0 | 1,92E+09 |
| capsules humides E1 | 0 | 1,10E+08 |
| capsules humides E2 | 0 | 1,24E+08 |
| temoin negatif | 10 | 0,00E+00 |
| phage libre | 10 | LDD |
| capsules humides E1 | 10 | 9,73E+06 |
| capsules humides E2 | 10 | 1,04E+07 |
| temoin negatif | 20 | 0,00E+00 |
| phage libre | 20 | LDD |
| capsules humides E1 | 20 | 3,52E+06 |
| capsules humides E2 | 20 | NA |
| temoin negatif | 30 | 0,00E+00 |
| phage libre | 30 | LDD |
| capsules humides E1 | 30 | 3,82E+06 |
| capsules humides E2 | 30 | 2,73E+06 |

**[Tableau 8]**

| **echantillon** | **Temps d'exposition** | **Concentration (UFP/MI ou UFP/g)** |
|---|---|---|
| temoin negatif | 0 | 0,00E+00 |
| phage libre | 0 | 1,69E+09 |
| capsules seches E1 | 0 | 6,62E+08 |
| capsules seches E2 | 0 | 8,04E+07 |
| temoin negatif | 10 | 0,00E+00 |
| phage libre | 10 | 5,44E+04 |
| capsules seches E1 | 10 | 6,06E+08 |
| capsules seches E2 | 10 | 8,89E+07 |
| temoin negatif | 20 | 0,00E+00 |
| phage libre | 20 | LDD |
| capsules seches E1 | 20 | 8,56E+07 |
| capsules seches E2 | 20 | 7,20E+08 |
| temoin negatif | 30 | 0,00E+00 |
| phage libre | 30 | LDD |
| capsules seches E1 | 30 | 1,11E+08 |
| capsules seches E2 | 30 | 5,88E+07 |

Les Tableaux 9 et 10 présentent les résultats de titration des phages 31 Dx après des temps d'exposition aux UV respectivement de 0, 5, 10, 20 et 30 minutes. LDD signifie limite de détection, respectivement pour les capsules humides et les capsules sèches.

**[Tableau 9]**

| **echantillon** | **Temps d'exposition** | **Concentration (UFP/MI ou UFP/g)** |
|---|---|---|
| temoin negatif | 0 | 0,00E+00 |
| phage libre | 0 | 1,72E+07 |
| capsules humides E1 | 0 | 2,41E+06 |
| capsules humides E2 | 0 | 1,18E+06 |
| temoin negatif | 10 | 0,00E+00 |
| phage libre | 10 | LDD |
| capsules humides E1 | 10 | 4,09E+06 |
| capsules humides E2 | 10 | 5,06E+05 |
| temoin negatif | 20 | 0,00E+00 |
| phage libre | 20 | LDD |
| capsules humides E1 | 20 | 6,57E+04 |
| capsules humides E2 | 20 | 7,23E+04 |
| temoin negatif | 30 | 0,00E+00 |
| phage libre | 30 | LDD |
| capsules humides E1 | 30 | 1,71E+05 |
| capsules humides E2 | 30 | 2,96E+05 |

**[Tableau 10]**

| **echantillon** | **Temps d'exposition** | **Concentration (UFP/MI ou UFP/g)** |
|---|---|---|
| temoin negatif | 0 | 0,00E+00 |
| phage libre | 0 | 5,83E+06 |
| capsules seches E1 | 0 | 5,14E+06 |
| capsules seches E2 | 0 | 4,21E+06 |
| temoin negatif | 10 | 0,00E+00 |
| phage libre | 10 | LDD |
| capsules seches E1 | 10 | 1,75E+06 |
| capsules seches E2 | 10 | 5,65E+06 |
| temoin negatif | 20 | 0,00E+00 |
| phage libre | 20 | LDD |
| capsules seches E1 | 20 | 3,39E+06 |
| capsules seches E2 | 20 | 1,58E+06 |
| temoin negatif | 30 | 0,00E+00 |
| phage libre | 30 | LDD |
| capsules seches E1 | 30 | 1,22E+06 |
| capsules seches E2 | 30 | 6,44E+06 |

### Exemple 3 : protection d'un cocktail de phages dans une capsule comprenant un filtre anti-UV.

Un cocktail de phages dirigés contre les bactéries *Salmonella,* Salmofresh^{™} commercialisé par Intralytix Inc. a été encapsulé dans des capsules comprenant un filtre anti-UV. Parallèlement, deux phages individuels composant ledit cocktail, respectivement le phage SBA 1781 et le phage SPT, ont été encapsulés dans le même type de capsules. Tous ces phages ont été exposés aux UV selon le protocole indiqué *supra* et dénombrés, en comparaison avec le cocktail de phages ou le phage libre et un témoin négatif.

Cette expérience a été effectuée à la fois avec des capsules humides et des capsules sèches.

Les résultats sont présentés dans les Tableaux 11 et 12 ci-dessous.

**[Tableau 11]**

| **Salmofresh^{™}** | | | | |
|---|---|---|---|---|
| **tps expo (min)** | **temoin neg** | **phage libre** | **CH E1** | **CH E2** |
| 0 | LDD | 5,69E+07 | 9,20E+05 | 7,80E+05 |
| 10 | LDD | 4,20E+03 | 2,61E+05 | 3,44E+05 |
| 20 | LDD | 9,33E+01 | 1,48E+05 | 9,10E+04 |
| 30 | LDD | LDD | 9,20E+04 | 2,01E+05 |

| **SBA 1781** | | | | |
|---|---|---|---|---|
| **tps expo (min)** | temoin neg | phage libre | **CH E1** | **CH E2** |
| 0 | LDD | 1,96E+05 | 2,80E+02 | 2,10E+02 |
| 10 | LDD | LDD | 2,80E+02 | 1,70E+02 |
| 20 | LDD | LDD | 7,00E+01 | 4,00E+01 |
| 30 | LDD | LDD | 2,00E+01 | 1,00E+02 |

| SPT1 | | | | |
|---|---|---|---|---|
| **tps expo (min)** | **temoin neg** | **phage libre** | **CH E1** | **CH E2** |
| 0 | LDD | 2,10E+07 | 4,40E+05 | 3,60E+05 |
| 10 | LDD | LDD | 1,68E+05 | 1,78E+05 |
| 20 | LDD | LDD | 2,41E+05 | 2,18E+05 |
| 30 | LDD | LDD | 1,27E+05 | 9,50E+04 |

**[Tableau 12]**

| **Salmofresh^{™}** | | | | |
|---|---|---|---|---|
| **tps expo (min)** | **temoin neg** | **phage libre** | **CS E1** | **CS E2** |
| 0 | LDD | 4,90E+07 | 3,50E+05 | 4,00E+05 |
| 10 | LDD | 9,10E+03 | 1,25E+05 | 1,38E+05 |
| 20 | LDD | 3,27E+02 | 1,71E+05 | 1,62E+05 |
| 30 | LDD | 4,67E+01 | 6,50E+04 | 6,90E+04 |

| **SBA 1781** | | | | |
|---|---|---|---|---|
| **tps expo (min)** | **temoin neg** | **phage libre** | **CS E1** | **CS E2** |
| 0 | LDD | 3,31E+04 | 1,10E+02 | 1,00E+02 |
| 10 | LDD | LDD | 1,50E+02 | 7,00E+01 |
| 20 | LDD | LDD | 6,00E+01 | 7,00E+01 |
| 30 | LDD | LDD | 1,10E+02 | 1,10E+02 |

| **SPT1** | | | | |
|---|---|---|---|---|
| **tps expo (min)** | **temoin neg** | **phage libre** | **CS E1** | **CS E2** |
| 0 | LDD | 1,68E+07 | 3,70E+04 | 3,60E+04 |
| 10 | LDD | 1,40E+02 | 7,60E+04 | 2,30E+04 |
| 20 | LDD | LDD | 3,60E+04 | 1,80E+04 |
| 30 | LDD | LDD | 2,90E+04 | 3,90E+04 |

Ces résultats montrent une diminution très importante de la quantité de phages dans le témoin phage libre (c'est-à-dire non encapsulé) dès 10 minutes d'exposition aux UV et une viabilité préservée par les capsules comprenant un filtre UV. Ladite viabilité étant démontrée via l'activité antibactérienne des phages contre leur bactérie cible, ces résultats démontrent la stabilité de la composition antibactérienne selon l'invention.

## Revendications

1. Composition antibactérienne comprenant :
au moins un bactériophage choisi parmi les *Podoviridae,* les *Myoviridae* et les *Siphoviridae,* à titre d'agent actif, dans un cœur liquide,
une coque gélifiée encapsulant le cœur liquide comprenant ledit bactériophage et comprenant des particules colloïdales de filtre UV dispersées dans laquelle le filtre UV n'est pas un composé soluble antioxydant.

2. Composition antibactérienne selon la revendication 1 dans laquelle le filtre UV est choisi parmi l'oxyde de titane, le noir de carbone, le biochar, le charbon de bois, le latex, la silice, les argiles, et leurs mélanges.

3. Composition selon l'une des revendications 1 ou 2 dans laquelle le bactériophage cible au moins une bactérie pathogène de plante.

4. Composition selon la revendication 3 dans laquelle ladite au moins une bactérie pathogène de plante est choisie parmi *Erwinia spp., Xanthomonas spp., Xylella spp., Pseudomonas spp., Ralstonia spp.*

5. Composition selon la revendication 4 dans laquelle ladite bactérie pathogène de plante est du genre *Erwinia.*

6. Composition selon l'une des revendications 1 ou 2 dans laquelle le bactériophage cible au moins une bactérie pathogène susceptible d'infecter les humains, préférentiellement choisie parmi *Escherichia coli, Listeria monocytogenes, Campylobacter jejuni, Staphylococcus aureus, Clostridium perfringens* ou une bactérie du genre *Salmonella.*

7. Composition selon l'une des revendications 1 ou 2 dans laquelle le bactériophage cible au moins une bactérie pathogène des animaux, préférentiellement choisie parmi *Escherichia coli, Salmonella, Campylobacter, Staphylococcus, Flavobacterium, Yersinia, Clostridium.*

8. Composition selon l'une des revendications 1 ou 2 dans laquelle le bactériophage cible au moins une bactérie pathogène susceptible de contaminer des produits de l'industrie agroalimentaire, ladite bactérie étant préférentiellement choisie parmi *Listeria, Salmonella, Shigella, Escherichia coli, Campylobacter jejuni.*

9. Utilisation d'une composition antibactérienne selon l'une quelconque des revendications 1 à 5 pour le traitement de cultures végétales, de leurs semences ou des produits de récoltes.

10. Utilisation d'une composition antibactérienne selon l'une quelconque des revendications 1 à 5 pour le traitement des sols.

11. Procédé de prévention et/ou de traitement de plants végétaux contre au moins une bactérie pathogène comprenant les étapes de : fournir une composition antibactérienne comprenant au moins un bactériophage au titre d'agent actif contre ladite au moins une bactérie pathogène, ledit bactériophage étant protégé contre les UV dans le cœur d'une microcapsule comprenant des particules colloïdales de filtre UV dispersées dans la coque encapsulant le coeur, dans laquelle le filtre UV n'est pas un composé soluble antioxydant,
appliquer ladite composition sur les parties aériennes du plant végétal et/ou par épandage sur le sol, ou autour des semences.

12. Utilisation d'une composition antibactérienne selon l'une des revendications 1, 2 ou 6 pour la nutrition et/ou l'alimentation humaine.

13. Utilisation d'une composition antibactérienne selon l'une des revendications 1, 2 ou 7 pour la nutrition et/ou l'alimentation animale.

14. Composition pharmaceutique comprenant une composition antibactérienne selon l'une des revendications 1 ou 2.

15. Composition selon la revendication 14 pour son utilisation pour le traitement de mammifères et de préférence d'humains.
